# NEW EUROPEAN PATENT SPECIFICATION

(11) **EP 1 719 546 B2**
(45) Date of publication and mention of the opposition decision: **13.07.2016**
(45) Mention of the grant of the patent: 15.12.2010
(21) Application number: 05009760.9
(22) Date of filing: 04.05.2005
(51) Int. Cl.: A61Q 5/12, A61K 8/34, A61K 8/39, A61K 8/41, A61K 8/73, A61K 8/86, A61K 8/891

(54) **Hair conditioning composition**
Haarkonditionierungszusammensetzung
Composition de soin capillaire

(43) Date of publication of application: 08.11.2006
(73) Proprietor: Kao Germany GmbH, 64297 Darmstadt (DE)
(72) Inventor: Hermes, Frank, 64342 Seehelm-Jugenheim (DE)

(56) References cited:
- EP-A- 1 531 007
- EP-A- 1 570 833
- WO-A-01/43859
- WO-A1-2006/000257
- FR-A- 2 712 490
- US-A- 4 725 433
- US-A- 5 641 480
- US-A- 5 942 216
- US-A1- 2002 197 213
- US-A1- 2003 134 760
- US-B1- 6 231 844
- US-B1- 6 274 128
- US-B1- 6 635 702

## Description

The present invention relates to a process for a conditioning hair having excellent volume and body enhancing effect.

Hair conditioners of various types either in application or in the form of preparation have found their wide range of usage in hair dressing practice. Among those rinse off types of conditioners have always been preferred in daily hair care. Usually rinse off type of conditioners are in the form of emulsions and comprising fatty alcohols and emulsifiers of different ionic characters as the main principal ingredients. In addition, they comprise conditioning ingredients of various types and especially those of cationic surfactants and/or cationic polymers. A general overview on the known hair conditioning products and also their usual compositions can be found in the monography of K. Schrader, "Grundlage und Rezepturen der Kozmetika", 2nd Ed. 1989, pp 722 - 781.

Although the state of the art is quite well advanced, some of the problems with conditioners have not yet been well addressed or solved. One of them is variability of conditioning properties of conventional conditioners depending on the hair type, i.e. damaged, fine, thick, normal, healthy hair types. As conditioners are used mainly for detangling and/or softening hair, the mainly used cationic compounds are deposited onto hair surface for achieving those effects, They usually make hair heavy and therefore cause volume down effect (loss of volume by making hair "heavy"), although the surface conditioning properties are excellent. Without making a rule, conditioners are generally designed for different hair types depending on their damage levels. On the other hand, conditioners are highly desirable designed for different hair strengths depending on for example hair diameter as defined such as coarse and fine hair. Although the big efforts, volume reducing effect of conditioners has not been overcome satisfactorily and conditioning compositions improving volume and body of hair are highly desirable.

US 2002/0197213 A1 discloses composition for a hair treatment preparation in the form of an aerosol foam comprising at least one non-ionic, amphiphilic associative thickener such as polyether-1 for increasing volume of hair. Document mentions addition of ethoxylated fatty alcohols as an option and uses Laureth-4 in some of the examples. However, there is no mention of volumizing and bodyfying effects of combination of fatty alcohol ethoxylates.

Thus, the objective of the present application is to find out a conditioner composition, which does not show above mentioned disadvantageous and excellently suitable for using in rinse-off application and enhances hair body and volume. It should be noted that the volume down problem with the conventional conditioners is an especially aggravated problem with fine hair so that conditioner compositions of the present invention are especially suitable for conditioning fine hair.

It has surprisingly been found out that a conditioner composition comprising at least one hair conditioning compound and at least two ethoxylated fatty alcohols with alkyl chain length of 10 to 22 C atoms, one having up to 3 ethoxy groups and the other having 4 to 20 ethoxy groups show excellent volumizing and bodyfying effects. The bodfying and volumizing effects are found to be synergistic as no such effect is observed with compositions comprising only one of the ethoxylated fatty alcohol.

The object of the present invention is a process for increasing volume and body of hair wherein a composition comprising in a cosmetically acceptable medium, at least one hair conditioning compound, at least one ethoxylated fatty alcohol with 1 to 3 ethoxy units and a second ethoxylated fatty alcohol with 4 to 20, preferably 4 to 10, more preferably 4 to 6 ethoxy units at a total concentration of first and second ethoxylated fatty alcohols of 0.5 to 10% by weight, with the condition that first and second ethoxylated fatty alcohols have an alkyl chain length of 10 to 22 C atoms, wherein the first and second ethoxylated fatty alcohols are present at a weight ratio of 5:1 to 1:5. is applied onto shampooed, preferably freshly, hair-and left on the hair for 1 to 20 min, preferably 1 to 15 min and most preferably 1 to 10 min and rinsed of with water.

The conditioners according to the process of the present invention improve additionally combability, shine and elasticity of hair.

The ethoxylated fatty alcohols having 10 to 22, preferably 10 to 18, more preferably 10 to 16 and most preferably 12 to 14 carbon atoms in the alkyl chain are useful for the compositions of the present invention according to the process.

Examples of the suitable ethoxylated fatty alcohols are as the first compound laureth-1 to 3, myreth 2 and 3, ceteth 1 to 3, steareth 2 and 3, and ceteareth 2 and 3, and as the second compound Laureth-4 to 20, myreth 5 to 10, ceteth 4 to 20, steareth 4 to 20, and ceteareth 4 to 20. The most preferred fatty alcohol is the lauryl alcohol and accordingly, the preferred first ethoxylated fatty alcohol is Laureth-2, and the second ethoxylated fatty alcohol is Laureth-4.

Total concentration of the ethoxylated fatty alcohols is in the range of 0.5 to 10%, preferably 0.5 to 7.5% and more preferably 0.5 to 5% and most preferably 0.5 to 3% by weight. The ratio of the two types of ethoxylated fatty alcohols is in the range of 5:1 to 1:5, more preferably 3:1 to 1: 1 and most preferably 1:1, by weight.

Without making a rule, it Is the preferred embodiment of the present invention that conditioning composition comprises the two ethoxylated fatty alcohol compounds with the same fatty alkyl chain length, i.e. lauryl, myristyl, behenyl, stearyl, cetyl.

Conditioning compositions according to the process of the present invention comprise at least one hair conditioning compound. Suitable and the preferred ones are those of cationic compounds, especially cationic polymers and cationic surfactants. The most preferred conditioning compounds are cationic surfactants. Olly substances and non-ionic substances can as well be suitable used as conditioners in the compositions according to the process of the present invention.

Suitable cationic polymers are those of best known with their CTFA category name Polyquatemium. Typical examples of those Polyquatemium 6, Polyquatemium 7, Polyquatemium 10, Polyquatemium 11, Polyquatemium 16, Polyquatemium 22, Polyquatemium 28 and Polyquatemium 37.

As well those polymers known with their CTFA category name Quatemium are suitable. Those are for example Quatemium-8, Quatemium-14, Quatemium-15, Quatemium-18, Quatemium-22, Quatemium-24, Quatemium-26, Quatemium-27, Quatemium-30, Quatemium-33, Quatemium-53, Quatemium-60, Quatemium-61, Quatemium-72, Quatemium-78, Quatemium-80, Quatemium-81, Quatemium-81, Quatemium-82, Quatemium-83 and Quatemium-84.

It has further been found out that especially those of cationic cellulose type polymers known as Polymer JR type from Amerchol such as Polyquatemium 10 or cationic guar gum known with trade name Jaguar from Rhône-Poulenc and chemically for example Guar hydroxyprvpyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquatemium 6 and polyquatemium 7.

The cationic polymers also include the quatemized products of graft polymers from organopolysiloxanes and polyethyl oxazolines described in EP-A 524 612 and EP-A 640 643.

Typical concentration range for cationic polymers can be 0.01- 5% by weight, preferably 0.01 - 3.5% by weight, more preferably 0.05 - 2.5% and most preferably 0.1 -1.5% by weight calculated to the total composition.

In the most preferred form of the process according to the present invention, conditioning compositions comprise at least one cationic surfactant as conditioning agent and as well as emulsifier. Suitable cationic surfactants are presented with the general formula 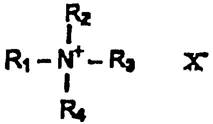 where R₁ is a saturated or unsaturated, branched or non-branched alkyl chain with 8-22 C atoms or

R₅ CO NH (CH₂)ₙ

where R₆ is saturated or unsaturated, branched or non-branched alkyl chain with 7-21 C atoms and n has value of 1 - 4, or

R₆ CO O (CH₂)n

where R₆ is saturated or unsaturated, branched or non-branched alkyl chain with 7-21 C atoms and n has value of 1-4, and R₂ is H or unsaturated or saturated, branched or non-branched alkyl chain with 1 - 4 C atoms or

R₅ CO NH (CH₂)ₙ

or

R₆ CO O (CH₂)ₙ

where R₅, R₆ and n are same as above.

R₃ and R₄ are H or lower alkyl chain with 1 to 4 carbon atoms, and X is anion such as chloride, bromide, methosulfate.

Typical examples of those ingredients are cetyl trimethyl ammonium chloride, stearyl trimonium chloride, behentrimonium chloride, stearamidopropyl trimonium chloride, cocamidopropyl trimonium chloride, dioleoylethyl dimethyl ammonium methosulfate, dioleoylethyl hydroxyethylmonium methosutfate.

From the above quaternary ammonium compounds disclosed with the general formula, especially to mention are those compounds known per se and are on the market, for example, under the trade names "Schercoquat^{®}", "Dehyquart^{®} F30" and "Tetranyl^{®}". Use of these compounds, the so-called "esterquats", In hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to mention are those compounds are known per se and on the market, for example, under the trade name "INCROQUAT^{®} HO" or "OCS". Those compounds known with a general ingredient category under "amidoquat" in the cosmetic industry.

Typical concentration range for cationic surfactants can be 0.01 - 5% by weight, preferably 0.01 - 3.5% by weight, more preferably 0.05 - 2.5% and most preferably 0.1 - 1.5% by weight calculated to the total composition.

It is a further preferred embodiment of the invention, that the cationic surfactants and the cationic polymers are used in combination in principal at any ratio, preferably at a ratio in the range of 3:1 - 1:3, more preferably 2:1 - 1:2 and most preferably 1:1, all ratio's are by weight

Oily substances are selected from such as silicone oils, either volatile or non-volatile, natural and synthetic oils. Among silicone oils those can be added to the compositions include dimethicone, amodimethicone (a cationic silicone), dimethiconol, polydimethylsiloxane, DC fluid ranges from Dow Coming, natural oils such as olive oil, almond oil, avocado oil, weizenkeim oil, ricinus oil and the synthetic oils, such as mineral oil, isopropyl myristate, palmitate, stearate and isostearate, oleyl oleate, Isocetyl stearate, hexyl laurate, dibutyl adipate, dioctyl adipate, myristyl myristate and oleyl erucate.

Non-ionic conditioning agents may be polyols such as glycerin, glycol and derivatives, poiyethyleneglycoles known with trade names Carbowax PEG from Union Carbide and Polyox WSR range from Amerchol, polyglycerin, polyethyleneglycol mono or di fatty acid esters having general formula

R₇CO(OCH₂CH₂)ₙOH

R₇CO(OCH₂CH₂)ₙOOCR₈

where R₇ and R₈ are independent from each other saturated, unsaturated or branched or non-branched alkyl chain with 7 to 21 C atoms and n is typically 2 - 100.

The compositions according to the process of the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R"}_{.}

In principal, the conditioning compositions according to the process of the present invention can be in any product form such as emulsions, gels, solutions, dispersions, aerorols. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

However, it is a further preferred embodiment according to the process of the present invention that the conditioning compositions are in the form of emulsion. The emulsion type of conditioners comprise additionally at least one fatty alcohol with alkyl chain length of 8 - 24 C atoms. Concentration of fatty alcohols is usually less than 20%, preferably less than 15% by weight calculated to total composition. More preferably the concentration of one or more fatty alcohols is in the range of 0.5 to 15% by weight, most preferably 0.5 to 10% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol and it is the most preferred fatty alcohol mixture in the compositions accordidng to the process of the present invention.

According to the process of the invention, conditioning composition can comprise at least one direct acting dyestuff and be used as conditioning and colouring composition. The direct dyes referred are cationic, anionic, neutral and of plant dyes.

Suitable cationic dyestuffs are in principal those available on the market for hair colouring applications. Some examples to those are: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

For this purpose, special reference is made to the PCT application WO 95/15144 of Ciba-Geigy AG. The cationic dyestuffs with the following chemical structures are especially preferred ones according to the process of the present invention. 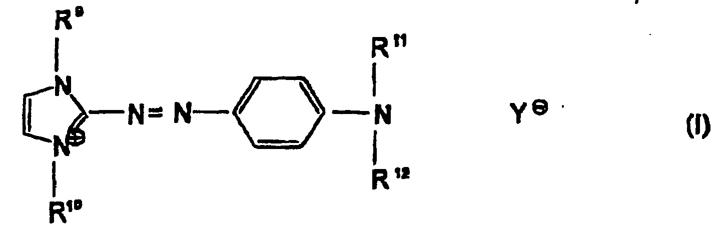 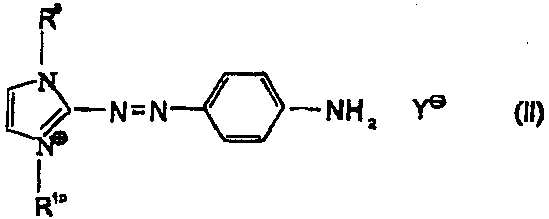 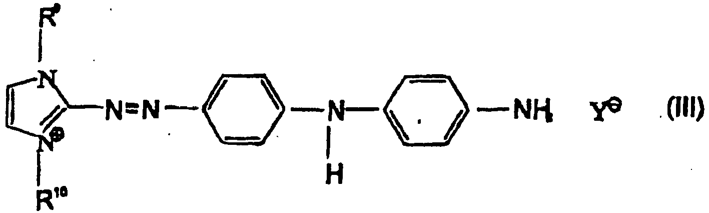 and 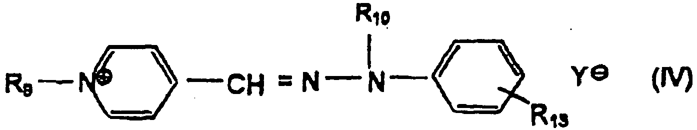 wherein R⁹, R¹⁰, R¹¹ and R¹² stand for hydrogen, a CH₃- or C₂H₅- group, R¹³ stands for hydrogen, -OCH₃ or -OC₂H₅ and Y Is an anion such as chloride, bromide, methosulfate.

The most preferred cationic dyestuffs are the ones according to the formula I, where R₁, R₂, R₃ and R₄ are methyl and Y is chloride, according to formula 11 where R₁ and R₂ are methyl and Y is chloride and according to the formula IV, where R₁ and R₂, are methyl, R₅ is hydrogen and Y is methosulfate.

Cationic dyestuffs can be included into the compositions according to the process of the present invention at a concentration of 0,001 to 2%, preferably 0.005 to 1.5% and more preferably 0.01 to 1% by weight, calculated to total aqueous composition.

Anionic dyes may as well be used either alone or in combination with cationic direct dyes at minor quantities. The suitable ones are:

Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D$C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

According to the invention, anionic dyes may be included at a concentration of 0.001 to 2%, preferably 0.005 to 1.5% and more preferably 0.01 to 1% by weight calculated to total aqueous composition.

Additionally, the coloring compositions according to the process the present invention may comprise neutral dyes (HC dyes), so called nitro dyes either alone or in addition to the cationic and/or anionic direct dyes. Concentration of those can typically be in the range of 0.001 to 1%, preferably 0.01 to 0.75% and more preferably 0.05 to 0.5% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8. HC Red No.9, HC Red No. 10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used in combination with cationic direct-acting dyestuffs, for example henna (red or black), alkanna root, laccaic acid, indigo, logwood powder, madder root and rhubarb powder.

Compositions according to the process of the present invention can comprise UV filters for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-meth-oxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-ldenebomane-2-one and the salts thereof and/or 3-(4'-methyl benzylidene)-DL-campher.

The preferred amount of the UV-absorber ranges from 0.01 % to 2.5%, more preferably from 0.05 % to 1 % by weight, calculated to the total composition.

The conditioner composition can contain active ingredients selected from moisturisers, sequestering agents, hair restructuring agents and natural ingredients showing conditioning benefits.

The compositions according to the process of the present invention can comprise hair-restructuring agents. The hair restructuring agents preferred are especially the ones disclosed in the German patent DE 197 51 550 C2.

One of the known hair restructuring agents is ceramide type of compound with the general formula 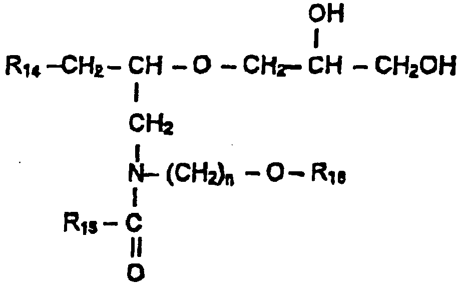 where R¹⁴ and R¹⁵ are independent from each other alkyl- or, alkenyl group with 10 to 22 carbon atoms, R¹⁶ is methyl, ethyl, n-propyl or isopropyl group and n is a number between 1 to 6, preferably 2 or 3,

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions according to the process of the present invention can be in the range of 0.01 to 2% and especially 0.01 to 1 % by weight calculated to the total weight of the composition. The fatty acids may be contained at a level of 0.01 to 2.5% and especially 0.01 to 1 % by weight calculated to the total weight of the composition. Phytosterol concentration of the conditioners is less than 1% and preferably less than 0.5% by weight calculated to the total weight of the composition. It should be noted without limiting the use of those ingredients that the effect of those hair restructuring ingredients Is especially elevated when used in combination with penetration enhancers.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is from 0.01 - 2.5% by weight calculated to the total composition.

Conditioner compositions according to the processof of the present invention can optionally contain non-ionic polymers such as hydroxyethylcellulose, hydroxypropylclellulose, xyloglucan, xanthan gum, polyvinylalcohol, polyvinylpyrrolidone or their derivatives.

The concentration of polymers is typically from 0.01 to 5%, preferably 0.05 - 3%, and more preferably 0.05 - 2% by weight, calculated to the total composition.

Conditioners according to the process of the present invention may comprise one or more surfactants as emulsifier and/or solubilizers other than those fatty alcohol ethoxylates. Suitable surfactants are of anionic, amphoteric and non-ionic type or their mixtures. Suitable non-ionic surfactants are compounds from the category of alkyl polyglucosides with the general formula

R₁₇-O-(CH₂CH₂O)ₙ-Zₓ,

wherein R₁₇ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Other additionally useful non-ionic surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R"}.

Hydrogenated castor oil with variable ethylene glycol units is found to be suitable non-ionic solubilizers. Those are for example known from BASF under the trade name Cremophor.

Further optional nonionic surfactant components are fatty acid mono- and dialkanolamides, such as coco fatty acid monoethanolamide and myristic fatty acid monoisopropanolamide.

Suitable anionic surfactants are of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sutfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the Invention are a-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁₈ - (C₂H₄O)ₙ - O - CH₂COOX,

wherein R₁₈ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 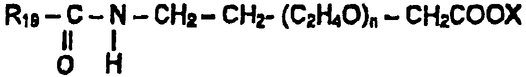 wherein R₁₉ and X have the above meanings, and n is in particular a number from 1 to 10, preferably 2.5 to 5. Such products have been known for some time and are on the market, for example, under the trade name "AKYPO^{®}" and "AKYPO-SOFT^{®}".

Further as the surfactants amphoteric or zwitterionic ones may be included in the conditioning compositions. The examples to mention are those of betaine, amido betaine, sulfobetaine or amido sulfobetaines. Specific examples are coco betaine, cocamidopropyl betaine, coco amphoacetate, cocamidopropyl sultaine.

The concentration of surfactants other than those of ethoxylated fatty alcohols may be typically from 0.01 to 10%, preferably 0.1- 7.5%, and more preferably 0.2 - 5% by weight, calculated to the total composition.

Natural plant extracts showing hair conditioning and/or restructuring effects can be used in the conditioners. Those are preferably the extracts from almond, Aloe Vera, coconut, mango, peach, lemon, wheat, rosemary, apricot, algae, grapefruit, sandalwood, lime and orange. Those extracts used are the ones commercially available and generally include organic solvents such as propylene glycol, butylenes glycol, ethanol, isopropanol. The active matter in those extracts can vary largely, i.e. in the range of 1 - 30% by weight. Concentration of those as an extract depending on the compatibility with the other ingredients can be in the range of 0.1 to 5%, preferably 0.1 to 2% by weight calculated to the total composition.

The pH of the conditioners according to the process of the present invention varies from 2 to 6.5, particularly 2 to 5, more particularly 2.5 to 4.5, most preferably 2.5 to 4.0.

The pH of the conditioner composition is adjusted to the required pH by using alkaline solution such as sodium hydroxide, potassium hydroxide, triethanolamine. For adjusting the pH of the conditioner compositions, following ingredients can as well be used: Organic acids such as citric acid, lactic acid, tartaric acid, malic acid, maleic acid, fumaric acid, levulinic acid, butyric acid and hydroxybutyric acids, valeric acid, oxalic acid, succinic acid, mandelic acid, glycolic acid, glucuronic acid, propionic acid, salloylic acid or acetic acid or inorganic acids such as hydrochloric acid, phosphoric acid, sulphuric acid, nitric acid. Concentration of the organic and/or inorganic acids or their mixtures should be adjusted in a way that conditioner composition is so obtained has a pH value between 2 and 6.5.

The conditioner compositions may contain organic solvents, for example, as penetration enhancer for any active substance in compositions such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol. Concentration of organic solvents in the conditioner composition can be in the range from 0.1 to 20% by weight, preferably 0.5 to 15% by weight, and more preferably 1 to 15% by weight calculated to the total composition. The organic solvents may at the same time serve to solubilize ingredients, which are not readily soluble or dispersible in the conditioner composition.

The conditioner compositions can have viscosity values between 1,000 mPa.s to 100,000 mPa.s, preferably 1,000 mPa.s to 80,000 mPa.s and more preferably 1,000 mPa.s to 60,000 mPa.s measured at 20°C with Brookfield viscosimetre with, for example, Spindie 4 at 10 rpm. The viscosity values are read after 30 seconds from the start of the measurement. In the selection of the viscosity, special attention must be paid to the way of application and packaging to be used. For example in the case an aerosol type of product is targeted, the viscosity of the composition is preferably lower than around 500 mPa.s. measured with the above method or any other suitable method.

The following examples are to illustrate the invention, but not limiting it.

### Example 1

**Composition**

| | A | B | C | D |
|---|---|---|---|---|
| Panthenol | 0.50 | 0.50 | 0.50 | 0.50 |
| Dimethicone | 0.20 | 0.20 | 0.20 | 0.20 |
| Cetearyl alcohol | 5.00 | 5.00 | 5.00 | 5.00 |
| Cetrimonium chloride | 1.00 | 1.00 | 1.00 | 1.00 |
| Laureth-2 | - | - | 1.00 | 1.00 |
| Laureth-4 | - | 1.00 | - | 1.00 |
| | | | | |
| Fragrance | | q.s. | | |
| Preservative | | q.s. | | |
| Lactic acid/sodium hydroxide | | q.s. to pH 3.5 | | |
| Water | | to 100 | | |

Composition A, B and C are comparative compositions according to the process of the present invention and Composition D is the composition according to the process of the present invention. All four compositions are tested in a two week home use test As the cleansing compositions a shampoo for normal hair is used from the brand Goldwell. The volunteers aging 20 to 45, 10 in each group were selected from females have up to shoulder length hair and having declared themselves as having volume and/or body problem. The volunteers in each group were asked to answer questions after using the conditioner concerning their hair quality. The marking was 1 being the worst and 5 being the best Obviously a result around 3 is the sign of no change in hair quality. The results are presented in the table below. In the table only invention relevant points are taken.

**Table I: Results of the home use test of the example I**

| | | Composition | | | |
|---|---|---|---|---|---|
| | | A | B | C | D |
| Wet hair | Easy combing | 3.8 | 3.7 | 3.8 | 3.9 |
| | Smooth | 4.1 | 4.0 | 4.2 | 4.3 |
| | | | | | |
| Dry hair | Easy combing | 3.7 | 3.6 | 3.8 | 3.9 |
| | Softness | 3.6 | 3.6 | 3.5 | 4.1 |
| | Shine | 3.6 | 3.4 | 3.2 | 4.1 |
| | Body | 2.9 | 3.1 | 3.0 | 4.3 |
| | Volume | 2.8 | 2.9 | 3.0 | 4.4 |
| | Elasticity | 3.1 | 2.8 | 3.1 | 3.9 |
| | Liking | 2.9 | 3.1 | 2.9 | 4.4 |

From the above results it is clear that the composition according to the process of the present investion score significantly higher than those of comparative compositions A, B and C. As the higher scores for volume and body were only observed when both ethoxylated fatty alcohols are present in the composition, those effects are the result of synergism between the two components.

Similar results are obtained with the following examples according to the process of the present investion.

### Example 2

| | |
|---|---|
| Cetearyl alcohol | 7.00 |
| Behentrimonium chloride | 1.50 |
| Panthenol | 0.50 |
| Laureth 2 | 1.00 |
| Laureth 4 | 1.00 |
| Ceramide of the formula* | 0.10 |
| Behenic acid | 0.10 |
| Avocadin | 0.20 |
| Almond oil | 0.10 |
| Fragrance | q.s. |
| Preservative | q.s. |
| Malic acid/sodium hydroxide | q.s to pH 3.7 |
| Water | to 100 |
| * as given in the description R₁₄ is C₁₅H₃₁; R₁₅ is C₁₆H₃₃ and R₁₆ is CH₃ and n=2 | |

### Example 3

| | |
|---|---|
| Cetearyl alcohol | 7.00 |
| dioleoylethyl dimethyl ammonium methosulfate | 1.50 |
| Glycerin | 1.50 |
| Laureth 2 | 0.75 |
| Laureth 4 | 1.00 |
| Polyquaternium 10 | 0.75 |
| Almond oil | 0.10 |
| Fragrance | q.s. |
| Preservative | q.s. |
| Lactic acid/sodium hydroxide | q.s to pH 4.0 |
| Basic red 51 | 0.07 |
| Water | to 100 |

The use of the composition on a dark blonde hair at a tone level of 6, resulted in a intensive red shine. The hair had improved volume and body.

### Example 4

| | |
|---|---|
| Cetearyl alcohol | 7.00 |
| Stearamidopropyltrimethylammonium chloride | 0.75 |
| Glycerin | 1.50 |
| Laureth 2 | 0.75 |
| Laureth 4 | 1.00 |
| Polyquaternium 10 | 0.75 |
| Almond oil | 0.10 |
| Fragrance | q.s. |
| Preservative | q.s. |
| Lactic acid/sodium hydroxide | q.s to pH 4.0 |
| Basic Yellow 87 | 0.04 |
| Basic Red 51 | 0.005 |
| Water | to 100 |

The use of the composition on a light blonde hair at a tone level of 9, resulted in a intensive gold shine. The hair had improved volume and body.

### Example 5

| | |
|---|---|
| Cetearyl alcohol | 7.00 |
| Cetrlmonium chloride | 0.60 |
| Glycerin | 1.50 |
| Laureth 2 | 0.75 |
| Laureth 4 | 0.75 |
| Polyquaternium 10 | 0.60 |
| Fragrance | q.s. |
| Preservative | q.s. |
| Malic acid/sodium hydroxide | q.s to pH 3.6 |
| Benzophenone-3 | 0.10 |
| Water | to 100 |

With the use of the above composition hair volume and body was increased and it protected hair from sunlight / UV light from colour changes.

### Example 6

| | |
|---|---|
| Cetearyl alcohol | 5.00 |
| Behentrimonium chloride | 1.00 |
| Glycerin | 1.50 |
| Laureth 2 | 1.50 |
| Laureth 4 | 1.50 |
| Polyquaternium 10 | 0.50 |
| Fragrance | q.s. |
| Preservative | q.s. |
| Citric acid/sodium hydroxide | q.s to pH 3.6 |
| Benzophenone-4 | 0.15 |
| Water | to 100 |

The above formula gave hair volume and body and after usage excellent shine and smoothness were observed.

### Example 7

| | |
|---|---|
| Cetearyl alcohol | 5.00 |
| Behentrimonium chloride | 0.50 |
| Dimethicone | 0.50 |
| Laureth 2 | 1.50 |
| Laureth 4 | 1.50 |
| Guar hydroxypropyltrimonium chloride | 0.50 |
| Fragrance | q.s. |
| Preservative | q.s. |
| Citric acid/sodium hydroxide | q.s to pH 4.0 |
| Basic blue 99 | 0.05 |
| Basic yellow 57 | 0.04 |
| Basic brown 16 | 0.08 |
| Basich red 76 | 0.06 |
| Water | to 100 |

The above composition turned hair colour into intensive shiny brown (base dark blonde) and gave hair excellently volume and body.

## Claims

1. Process for increasing volume and body of hair **characterised in that** a conditioning composition comprising at least one hair conditioning compound in a cosmetically acceptable medium **characterised In that** it comprises additionally
a- at least one first ethoxylated fatty alcohol with 1 to 3 ethoxy groups, and
b- at least one second ethoxylated fatty alcohol with 4 to 20 ethoxy groups, at a total concentration of a and b 0.5 to of 10% by weight
with the condition that first and second ethoxylated fatty alcohols have an alkyl chain length of 10 to 22 carbon atoms, wherein the first and second ethoxylated fatty alcohols are present at a weight ratio of 5:1 to 1:5 is applied onto shampooed hair and left on the hair for 1 to 20 min and rinsed of with water.

2. Process according to claim 1 **characterized in that** the conditioning compound is a cationic surfactant according to formula 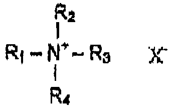
where R, is a saturated or unsaturated, branched or non-branched alkyl chain with 8-22 C atoms or
R₅CONH (CH₂)ₙ
where R_{5 i}s saturated or unsaturated. branched or non-branched alkyl chain with 7-21 C atoms and n has typical value of 1 - 4 or
R₆COO(CH₂)ₙ
where R₆ is saturated or unsaturated, branched or non-branched alkyl chain with 7-21 C atoms and n has typical value of 1 - 4, and
R₂ is a hydrogen, lower alkyl chain with 1 to 4 carbon atoms, saturated or unsaturated, branched or non-branched alkyl chain with 8-22 C atoms or
R₅CONH(CH₂).
where R₅ is saturated or unsaturated, branched or non-branched alkyl chain with 7-21 C atoms and n has typical value of 1 - 4 or
R₆COO(CH₂)ₙ
where R₆ is saturated or unsaturated, branched or non-branched alkyl chain with 7-21 C atoms and n has typical value of 1 - 4, and
R₃ and R₄ are independent from each other H or lower alkyl chain with 1 to 4 carbon atoms, and X is chloride, bromide or methosulfate.

3. Process according to claims 1 and 2 **characterized in that** the conditioning compound is a cationic polymer.

4. Process according to claims 4 to 3 **characterized in that** the conditioning compound is a silicone compound.

5. Process according to claims 1 to 4 **characterized in that** the composition comprises at least one direct dye.

6. Process according to claim 5 **characterized in that** the composition comprise as the direct dye at least one cationic direct dye.

7. Process according to claim 5 **characterized in that** the composition comprises as the direct dye at least one anionic direct dye.

8. Process according to claims 1 to 7 **characterized in that** the composition comprises at least one UV filter.

9. Process according to claims 1 to 8 **characterized in that** the composition comprises at least one organic solvent.

10. Process according to claims 1 to 9 **characterized in that** the composition comprises at least one fatty alcohol with 8 to 24 C.

11. Process the composition according to claims 1 to 10 **characterised in that** the composition further comprises active ingredients selected from hair restructuring ingredients, moisturising ingredients, sequestering agents and natural plant extracts.

12. Process according to claims 1 to 11 **characterised in that** the composition has a pH in the range of 2 to 6.5.

## Patentansprüche

1. Verfahren zur Erhöhung von Volumen und Körper von Haaren, **dadurch gekennzeichnet, dass** eine konditionierende Zusammensetzung A die mindestens eine Haar konditionierende Verbindung in einem kosmetisch akzeptablen Medium enthält, **dadurch gekennzeichnet, dass** sie zusätzlich
a- Mindestens einen ersten ethoxylierten Fettalkohol mit 1 bis 3 Ethoxy- Gruppen, und
b- mindestens einen zweiten ethoxylierten Fettalkohol mit 4 bis 20 EthoxyGruppen in einer Gesamtmenge von a und b von 0,5 bis 10 Gew.- % enthält,
mit der Bedingung, dass erste und zweite ethoxylierte Fettalkohole eine Alkyl-Kettenlänge von 10 oder 22 Kohlenstoffatomen haben, worin die ersten- und zweiten ethoxylierten Fettalkohole in einem Gewichtsverhältnis von 5 : 1 bis 1 :5 vorliegen auf shampooniertes Haar aufgetragen wird, 1 bis 20 Minuten auf dem Haar bleibt und mit Wasser ausgespült wird.

2. Verfahren nach Anspruch 1, **dadurch gekennzeichnet, dass** die konditionierende Verbindung ein kationisches Tensid ist, nach der allgemeinen Formel 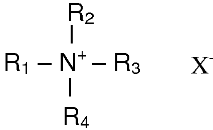 worin R₁ eine gesättigte oder ungesättigte, verzweigte oder nicht verzweigte Alkylkette mit 8 bis 22 C- Atomen ist, oder
R₅CONH(CH₂)n
worin R₅ eine gesättigte oder ungesättigte, verzweigte oder nicht verzweigte Alkylkette mit 7-21 C - Atomen ist und n einen typischen Wert von 1 - 4 hat, oder
R₆COO(CH₂)ₙ
worin R₆ eine gesättigte oder ungesättigte, verzweigte oder nicht verzweigte Alkylkette mit 7-21 C Atomen ist und n einen typischen Wert von 1 -4 hat, und R₂ ein Wasserstoff, eine niedrige Alkylkette mit 1 bis 4 Kohlenstoffatomen, eine gesättigte oder ungesättigte, verzweigte oder nicht verzeigte Alkylkette mit 8-22 C Atomen ist, oder
R₅CONH(CH₂)n
worin R₅ eine gesättigte oder ungesättigte, verzweigte oder nicht verzweigte Alkylkette mit 7-21 C Atomen ist und n einen typischen Wert von 1 - 4 hat, oder
R₆COO(CH₂)n
worin R₆ eine gesättigte oder ungesättigte, verzweigte oder nicht verzweigte Alkylkette mit 7-21 C Atomen ist und n einen typischen Wert von 1 - 4 hat, und
R₃ und R₄ unabhängig voneinander Wasserstoff oder eine niedrige Alkylkette mit 1 bis 4 Kohlenstoffatomen sind, und X Chlorid, Bromid oder Methosulfat ist.

3. Verfahren nach den Ansprüchen 1 und 2, **dadurch gekennzeichnet, dass** die konditionierende Verbindung ein kationisches Polymer ist.

4. Verfahren nach den Ansprüchen 1 bis 3, **dadurch gekennzeichnet, dass** die konditionierende Verbindung eine Silikonverbindung ist.

5. Verfahren nach den Ansprüchen 1 bis 4, **dadurch gekennzeichnet, dass** die Zusammensetzung mindestens eine direkt ziehende Farbe enthält.

6. Verfahren nach Anspruch 5, **dadurch gekennzeichnet, dass** die Zusammensetzung als direkt ziehende Farbe mindestens eine kationische direkt ziehende Farbe enthält.

7. Verfahren nach Anspruch 5, **dadurch gekennzeichnet, dass** die Zusammensetzung als direkt ziehende Farbe mindestens eine anionische direkt ziehende Farbe enthält.

8. Verfahren nach den Ansprüchen 1 bis 7, **dadurch gekennzeichnet, dass** die Zusammensetzung mindestens einen UV- Filter enthält.

9. Verfahren nach den Ansprüchen 1 bis 8, **dadurch gekennzeichnet, dass** die Zusammensetzung mindestens ein organisches Lösungsmittel enthält.

10. Verfahren nach den Ansprüchen 1 bis 9, **dadurch gekennzeichnet, dass** die Zusammensetzung mindestens einen Fettalkohol mit 8 bis 24 C- Atomen enthält.

11. Verfahren nach den Ansprüchen 1 bis 10, **dadurch gekennzeichnet, dass** die Zusammensetzung weiterhin aktive Inhaltsstoffe, ausgewählt aus Haar Restrukturierungsstoffen, Feuchtigkeitsspendern, Komplexbildnern und natürlichen Pflanzenextrakten, enthält.

12. Verfahren nach den Ansprüchen 1 bis 11, **dadurch gekennzeichnet, dass** die Zusammensetzung einen pH- Wert im Bereich von 2 bis 6,5 hat.

## Revendications

1. Procédé destiné à augmenter le volume et le corps des cheveux **caractérisé en ce qu'**une composition de soin A comprenant au moins un composé de soin capillaire dans un milieu acceptable sur le plan cosmétique **caractérisée en ce qu'**elle comprend en outre
c - au moins un premier alcool gras éthoxylé avec 1 à 3 groupes éthoxy, et
d - au moins un second alcool gras éthoxylé avec 4 à 20 groupes éthoxy, à une concentration totale en a et b de 0,5 à 10 % en poids
à condition que les premier et second alcools gras éthoxylés possèdent une longueur de chaîne d'alkyle de 10 ou 22 atomes de carbone, où les premier et second alcools gras éthoxylés sont présents à un rapport en poids de 5:1 à 1:5, est appliquée sur des cheveux shampouinés, puis on la laisse sur les cheveux pendant 1 à 20 min et on l'élimine par rinçage avec de l'eau.

2. Procédé selon la revendication 1, **caractérisé en ce que** le composé de soin est un tensioactif cationique selon la formule 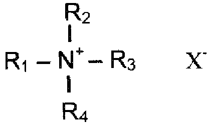 dans laquelle R₁ représente une chaîne d'alkyle saturée ou insaturée, ramifiée ou non ramifiée avec 8 à 22 atomes C ou
R₅ CO NH (CH₂)n
où R₅ représente une chaîne d'alkyle saturée ou insaturée, ramifiée ou non ramifiée avec 7 à 21 atomes C et n a une valeur typique de 1 à 4 ou
R₆COO(CH₂)n
où R₆ représente une chaîne d'alkyle saturée ou insaturée, ramifiée ou non ramifiée avec 7 à 21 atomes C et n a une valeur typique de 1 à 4, et
R₂ représente un atome d'hydrogène, une chaîne d'alkyle inférieur avec 1 à 4 atomes de carbone, une chaîne d'alkyle saturée ou insaturée, ramifiée ou non ramifiée avec 8 à 22 atomes C ou
R₅CONH(CH₂)n
où R₅ représente une chaîne d'alkyle saturée ou insaturée, ramifiée ou non ramifiée avec 7 à 21 atomes C et n a une valeur typique de 1 à 4 ou
R₆COO(CH₂)n
où R₆ représente une chaîne d'alkyle saturée ou insaturée, ramifiée ou non ramifiée avec 7 à 21 atomes C et n a une valeur typique de 1 à 4, et
R₃ et R₄ représentent, indépendamment l'un de l'autre, un atome H ou une chaîne d'alkyle inférieur avec 1 à 4 atomes de carbone, et X représente un chlorure, un bromure ou un méthosulfate.

3. Procédé selon les revendications 1 et 2, **caractérisé en ce que** le composé de soin est un polymère cationique.

4. Procédé selon les revendications 1 à 3, **caractérisé en ce que** le composé de soin est un composé siliconé.

5. Procédé selon les revendications 1 à 4, **caractérisé en ce que** la composition comprend au moins un colorant direct.

6. Procédé selon la revendication 5, **caractérisé en ce que** la composition comprend en tant que colorant direct au moins un colorant direct cationique.

7. Procédé selon la revendication 5, **caractérisé en ce que** la composition comprend en tant que colorant direct au moins un colorant direct anionique.

8. Procédé selon les revendications 1 à 7, **caractérisé en ce que** la composition comprend au moins un filtre U.V.

9. Procédé selon les revendications 1 à 8, **caractérisé en ce que** la composition comprend au moins un solvant organique.

10. Procédé selon les revendications 1 à 9, **caractérisé en ce que** la composition comprend au moins un alcool gras avec 2 à 24 atomes C.

11. Procédé selon les revendications 1 à 10, **caractérisé en ce que** la composition comprend de plus des ingrédients actifs choisis parmi des ingrédients restructurants pour cheveux, des ingrédients hydratants, des agents de séquestration et des extraits naturels de plante.

12. Procédé selon les revendications 1 à 11, **caractérisé en ce que** la composition possède un pH dans la gamme de 2 à 6,5.
